# EUROPÄISCHE PATENTANMELDUNG

(11) **EP 2 014 638 A2**
(43) Veröffentlichungstag der Anmeldung: **14.01.2009**
(21) Anmeldenummer: 08009587.0
(22) Anmeldetag: 27.05.2008
(51) Int. Cl.: C07C 37/70, C07C 39/16

(54) **Vermeidung von Feststoffablagerungen in Tropfenabscheidern durch Eindüsung von geeigneten Flüssigkeiten**

(30) Priorität: 08.06.2007 DE 102007026548
(71) Anmelder: Bayer MaterialScience AG, 51368 Leverkusen (DE)
(72) Erfinder: Münnich, Christian, 51377 Leverkusen (DE); Alps, Ernst-Joachim, 41539 Dormagen (DE); Westernacher, Stefan, 47906 Kempen (DE)

(57) **Zusammenfassung**

Die vorliegende Erfindung betrifft ein Verfahren zur Vermeidung von Feststoffablagerungen in Tropfenabscheidern (Demistern) durch Eindüsung von geeigneten Flüssigkeiten insbesondere bei der Herstellung von Bis(4-hydroxyaryl)alkanen.

## Beschreibung

Die vorliegende Erfindung betrifft ein Verfahren zur Vermeidung von Feststoffablagerungen in Tropfenabscheidern (Demistern) durch Eindüsung von geeigneten Flüssigkeiten bei der Herstellung von Bis(4-hydroxyaryl)alkanen.

Die Synthese von Bis(4-hydroxyaryl)alkanen durch sauer katalysierte Umsetzung aromatischer Hydroxyverbindungen mit Ketonen ist bekannt, beispielsweise aus US-A 2 775 620 oder EP-A 342 758. In der Regel wird als Zwischenprodukt ein Addukt des Bis(4-hydroxyaryl)alkans und der als Edukt eingesetzten aromatischen mono-Hydroxyverbindung erhalten, das anschließend destillativ wie z.B. in EP-A 343 349 oder EP-A 1 121 339 beschrieben von der aromatischen Hydroxyverbindung befreit wird.

Die Abtrennung der aromatischen mono-Hydroxyverbindung aus der Schmelze, auch "Strippen" genannt, vom Bis(4-hydroxyaryl)alkan ist literaturbekannt. In EP-A 343 349 wird beispielsweise mit Dampf in einer gepackten Kolonne bei 160 bis 200°C bei leicht reduziertem Druck die aromatische mono-Hydroxyverbindung vom Bis(4-hydroxyaryl)alkan abgetrennt. Die Nutzung von Dampf als Strippmedium kann zu Zersetzungsreaktionen des Bis(4-hydroxyaryl)alkans führen. Die so gebildeten Zersetzungsprodukte können sich auf die Qualität der aus den Bisphenolen hergestellten Produkte wie Epoxidharze, Polyester, Polyestercarbonate und Polycarbonate negativ auswirken, und zwar dergestalt, dass Farbprobleme, schlechte Lichtdurchlässigkeit transparenter Produkte oder Stippen in den Oberflächen von Formteilen aus diesen Endprodukten die Folge sind.

In EP-A 1 121 339 wird eine produktschonendere Abtrennung des Phenols durch ein Inertgas (Stickstoff), das im Vergleich zu Dampf zu weniger Zersetzung des Bis(4-hydroxyaryl)alkans führt, beschrieben. Der Stickstoff wird dabei nach der Abtrennung des Phenols durch Kondensation verdichtet, gewaschen und nach Vorwärmung wieder in den Desorber rezyklisiert.

Bei der Wäsche des verdichteten Stickstoffs mit Wasser verbleiben feine Tropfen der Waschflüssigkeit in diesem Gasstrom und können zu Belägen z. B. in dem nachgeschalteten Wärmetauscher führen, der zur Vorwärmung des Stickstoffs dient.

Daher kann in den Gasstrom zwischen der Wäsche und dem Wärmetauscher ein Demister eingesetzt werden, der die Funktion eines Tropfenabscheiders erfüllt und so Ablagerungen in den nachgeschalteten Anlagenteilen verhindert. Diese Abscheidung von Tropfen aus dem Gasstrom auf dem Demister ist aber mit einer Verstopfung des Demisters selbst durch Ablagerungen von phenolischen Bestandteilen verbunden, die aus der Waschflüssigkeit der Waschstufe oder einer nicht vollständigen Abscheidung im Waschschritt herrühren können. Diese Ablagerungen führen zur Verstopfung und bewirken eine Einschränkung der Funktionsfähigkeit des Demisters bis hin zu Produktionsausfällen durch Reinigungsmaßnahmen.

Demister als Tropfenabscheider sind bereits bekannt und beschrieben in "RÖMPP, Lexikon Chemie 2.0". Aus WO-A 2006/106582 ist ebenfalls bekannt, dass es bei der Abscheidung von (Meerwasser)Flüssigkeitstropfen aus Gasen auf Demistern zu Salzabscheidungen auf diesen Demistern kommen kann. Zur Vermeidung solcher Abscheidungen wird in der WO-A 2006/106582 vorgeschlagen, den Demister aus Leitblechen bestimmter Formgebung so zu konstruieren, dass geringfügig gebildete Ablagerungen auf diesen Leitblechen des Demisters durch ablaufende Tropfen wieder abgelöst werden. Ein solcher Demister ist eine Spezialkonstruktion und nicht handelsüblich. Zudem ist dessen einwandfreie Funktionsweise an bestimmte Gasstömungsgeschwindigkeiten gebunden, die es den Flüssigkeitstropfen erlauben, entgegen der Gasströmungsrichtung abzutropfen. Daher ist diese Maßnahme nicht ohne weiteres auf wechselnde Betriebbedingungen, z.B. wechselnde Strömungsbedingungen, anwendbar.

Es bestand demnach weiterhin Bedarf an einer einfachen Maßnahme zur Verhinderung von Feststoffabscheidungen sowohl in den Anlagenteilen zur Abtrennung der aromatischen mono-Hydroxyverbindung von Bis(4-hydroxyaryl)alkanen, wie z.B. Wärmetauschern für die Inertgasvorwärmung, als auch in den zur Gasreinigung verwendeten Demistern, welche die vorangehend genannten Nachteile nicht aufweist und einen möglichst langen störungsfreien kontinuierlichen Betrieb ermöglicht, so dass Produktionsausfälle durch Verstopfungen verhindert werden können.

Aufgabe der vorliegenden Erfindung war es daher, solche Maßnahmen bereitzustellen.

Vorteilhaft für möglichst einfache Maßnahmen ist dabei der Einsatz eines konventionellen Demisters, der bei wechselnden Betriebsbedingungen einsetztbar ist.

Überraschend wurde gefunden, dass Ablagerungen auf bzw. Verstopfungen des Demisters und der nachgeschalteten Anlagenteile sehr effizient vermieden werden können, wenn der handelsübliche Demister im bestimmungsgemäßen Betrieb der Anlage kontinuierlich auf der angeströmten Seite mit einer Waschlösung beaufschlagt wird, die aus dem Gasstrom abgeführt wird. Dadurch wird die Ablagerung von Feststoffen und damit die Verstopfungsgefahr des Demisters nicht nur sehr effizient vermieden sondern auch gegebenenfalls bereits gebildete Ablagerungen abgespült.

Gegenstand der vorliegenden Erfindung ist somit ein Verfahren zur Herstellung von Bis(4-hydroxyaryl)alkanen aus Addukten von Bis(4-hydroxyaryl)alkanen und aromatischen mono-Hydroxyverbindungen, bei dem
a) durch eine Schmelze eines Addukts aus einem Bis(4-hydroxyaryl)-alkan und einer aromatischen mono-Hydroxyverbindung bei 150°C bis 230°C ein Inertgas geleitet wird, wobei der Inertgasstrom die aromatische mono-Hydroxyverbindung aus der Schmelze entfernt,
b) die aromatische mono-Hydroxyverbindung aus dem Inertgasstrom durch Kondensation entfernt wird,
c) der Inertgasstrom
   aa) komprimiert, gereinigt und
   bb) durch wenigstens einen Demister geleitet wird, der mit einer Flüssigkeit beaufschlagt wird, und
   cc) wieder in Schritt a) zurückgeführt wird.

Das erfindungsgemäße Verfahren ist einsetzbar für die Aufarbeitung der Addukte von Bis(4-hydroxyaryl)alkanen und aromatischen mono-Hydroxyverbindungen.

Geeignete Bis(4-hydroxyaryl)alkane sind beispielsweise solche der allgemeinen Formel (I), 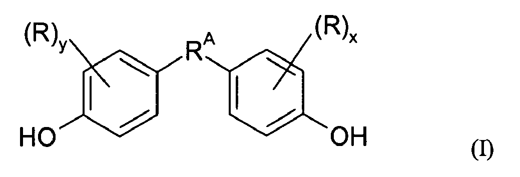 worin
- R^{A}: für einen linearen oder verzweigten C₁-C₁₈-Alkylenrest, bevorzugt C₁-C₆-Alkylenrest, oder einen C₅-C₁₈-Cycloalkylenrest, bevorzugt einen C₅-C₁₂-Cycloalkylenrest steht,
- R: unabhängig voneinander für einen linearen oder verzweigten C₁-C₁₈-Alkylrest, bevorzugt C₁-C₆-Alkylrest, einen C₅-C₁₈-Cycloalkylrest, bevorzugt einen C₅-C₁₂-Cycloalkylrest, einen C₆-C₂₄-Arylrest, bevorzugt einen C₆-C₁₂-Arylrest, oder einen Halogenrest stehen und
- x und y: unabhängig voneinander für 0 oder eine ganze Zahl von 1 bis 4, bevorzugt unabhängig voneinander für 0, 1 oder 2 stehen.

Bevorzugte Bis(4-hydroxyaryl)alkane sind 2,2-Bis-(4-hydroxyphenyl)propan (Bisphenol A (BPA)), 2,4-Bis-(4-hydroxyphenyl)-2-methylbutan, 2,2-Bis-(3-methyl-4-hydroxyphenyl)-propan, Bis-(3,5-dimethyl-4-hydroxyphenyl)-methan, 2,2-Bis-(3,5-dimethyl-4-hydroxyphenyl)-propan, 2,4-Bis-(3,5-dimethyl-4-hydroxyphenyl)-2-methylbutan, 1,1-Bis-(4-hydroxyphenyl)-cyclohexan und 1,1-Bis-(4-hydroxyphenyl)-3,3,5-trimethylcyclohexan (Bisphenol TMC).

Besonders bevorzugte Bis(4-hydroxyaryl)alkane sind 2,2-Bis-(4-hydroxyphenyl)-propan (Bisphenol A (BPA)), 2,2-Bis(3,5-dimethyl-4-hydroxyphenyl)-propan, 1,1-Bis-(4-hydroxyphenyl)-cyclohexan und 1,1-Bis-(4-hydroxyphenyl)-3,3,5-trimethylcyclohexan (Bisphenol TMC).

Ganz besonders bevorzugt ist 2,2-Bis-(4-hydroxyphenyl)-propan.

Bis(4-hydroxyaryl)alkane sind nach dem Fachmann bekannten Verfahren erhältlich durch Umsetzung von aromatischen mono-Hydroxyverbindungen, die in p-Position nicht substituiert sind, mit Ketonen, die wenigstens eine aliphatische Gruppe an der Carbonylfunktion aufweisen.

Geeignete aromatische mono-Hydroxyverbindungen sind beispielsweise solche der allgemeinen Formel (II), 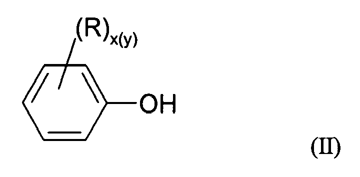 die in p-Position nicht substituiert sind und worin,
- R: unabhängig voneinander für einen linearen oder verzweigten C₁-C₁₈-Alkylrest, bevorzugt C₁-C₆-Alkylrest, einen C₅-C₁₈-Cycloalkylrest, bevorzugt einen C₅-C₁₂-Cycloalkylrest, einen C₆-C₂₄-Arylrest, bevorzugt einen C₆-C₁₂-Arylrest, oder einen Halogenrest stehen und
- x oder y: für 0 oder eine ganze Zahl von 1 bis 4, bevorzugt für 0, 1 oder 2 stehen.

Beispiele für geeignete aromatische mono-Hydroxyverbindungen der allgemeinen Formel (II) sind beispielsweise Phenol, o-und m-Kresol, 2,6-Dimethylphenol, o-tert.-Butylphenol, 2-Methyl-6-tert.-butylphenol, o-Cyclohexylphenol, o-Phenyl-phenol, o-Isopropylphenol, 2-Methyl-6-cyclopentyl-phenol, o-und m-Chlorphenol oder 2,3,6-Trimethylphenol. Bevorzugt sind Phenol, o-und m-Kresol, 2, 6-Dimethylphenol, o-tert.-Butylphenol und o-Phenyl-phenol, ganz besonders bevorzugt ist Phenol.

Geeignete Ketone sind beispielsweise solche der allgemeinen Formel (III), 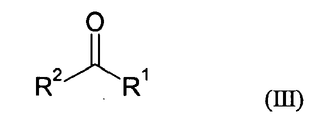 worin
- R¹: für einen linearen oder verzweigten C₁-C₁₈-Alkylrest, bevorzugt C₁-C₆-Alkylrest, steht und
- R²: für einen linearen oder verzweigten C₁-C₁₈-Alkylrest, bevorzugt C₁-C₆-Alkylrest, oder einen C₆-C₂₄-Arylrest, bevorzugt einen C₆-C₁₂-Arylrest, steht oder
- R¹ und R²: zusammen für einen linearen oder verzweigten C₄-C₁₈Alkylenrest, bevorzugt C₄-C₁₂-Alkylenrest stehen.

Beispiele für geeignete Ketone der allgemeinen Formel (III) sind Aceton, Methylethylketon, Methylpropylketon, Methylisopropylketon, Diethylketon, Acetophenon, Cyclohexanon, Cyclopentanon, Methyl-, Dimethyl- und Trimethylcyclohexanonen, die auch geminale Methylgruppen aufweisen können, z.B. 3,3-Dimethyl-5-methylcyclohexanon (Hydroisophoron). Bevorzugte Ketone sind Aceton, Acetophenon, Cyclohexanon und dessen Methylgruppen tragende Homologe, besonders bevorzugt ist Aceton.

**C₁-C₆-Alkyl** steht beispielsweise für Methyl, Ethyl, n-Propyl, iso-Propyl, n-Butyl, sec.-Butyl, tert.-Butyl, n-Pentyl, 1-Methylbutyl, 2-Methylbutyl, 3-Methylbutyl, neo-Pentyl, 1-Ethylpropyl, Cyclohexyl, Cyclopentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl oder 1-Ethyl-2-methylpropyl, **C₁-C₁₈-Alkyl** darüber hinaus beispielsweise für darüber hinaus beispielsweise für n-Heptyl und n-Octyl, Pinakyl, Adamantyl, die isomeren Menthyle, n-Nonyl, n-Decyl, n-Dodecyl, n-Tridecyl, n-Tetradecyl, n-Hexadecyl, n-Octadecyl oder Stearyl.

**C₁-C₆-Alkylen** bzw. **C₁-C₁₈-Alkylen** steht beispielsweise für die den vorangehenden Alkylgruppen entsprechenden Alkylengruppen.

**C₅-C₁₂-Cycloalkyl** steht beispielsweise für Cylopentyl, Cyclohexyl, Cyclooctyl oder Cyclododecyl.

Beispiele für **C₆-C₂₄-Aryl** oder **C₆-C₁₂-Aryl** sind Phenyl, o-, p-, m-Tolyl, Naphthyl, Phenanthrenyl, Anthracenyl oder Fluorenyl.

**Halogen** kann für Fluor, Chlor, Brom oder Iod, bevorzugt für Fluor oder Chlor, besonders bevorzugt für Chlor stehen.

Die im erfindungsgemäßen Verfahren eingesetzten Addukte aus einem Bis(4-hydroxyaryl)-alkan und einer aromatischen mono-Hydroxyverbindung ein halten als aromatische mono-Hydroxyverbindung die vorangehend bereits aufgeführten der allgemeinen Formel (II).

In einer bevorzugten Ausführungsform des erfindungsgemäßen Verfahrens wird als Addukts aus einem Bis(4-hydroxyaryl)-alkan und einer aromatischen mono-Hydroxyverbindung das Addukt von Bisphenol A (2,2-Bis-(4-hydroxyphenyl)-propan) und Phenol eingesetzt.

Die Abtrennung der aromatischen mono-Hydroxyverbindung vom Bis(4-hydroxyaryl)alkan aus der Schmelze des Addukts aus Bis(4-hydroxyaryl)alkan und aromatischer mono-Hydroxyverbindung erfolgt bei Temperaturen von 150°C bis 230°C, bevorzugt von 170°C bis 210°C. Dabei wird z.B. ein Inertgas oder Dampf durch die Schmelze des Addukts aus einem Bis(4-hydroxyaryl)-alkan und einer aromatischen mono-Hydroxyverbindung geleitet, wobei der Inertgasstrom die aromatische mono-Hydroxyverbindung aus der Schmelze austreibt. Dieser Vorgang wird im Folgenden auch als Strippen bezeichnet. Als Inertgas eignet sich beispielsweise Stickstoff, Kohlendioxid oder Edelgase, wie z.B. Argon. Bevorzugtes Inertgas ist Stickstoff. Als Dampf eignet sich beispielsweise Wasserdampf. Das Verhältnis von Inertgas oder Dampf zur Menge der Adduktschmelze liegt bevorzugt bei 10 m³ bis 1000 m³ Inertgas pro t Adduktschmelze. Das Strippen kann gegebenenfalls durch Verringerung des Druckes auf zwischen 0,1 und 1 bar_{absolut} in dem zur Abtrennung der aromatischen mono-Hydroxyverbindung eingesetzten Aggregat erleichtert werden, erfolgt aber bevorzugt unter Normaldruck oder leicht erhöhtem Druck, wie z.B. von 1 bis 2 bar_{absolut}. Das Strippen der aromatischen mono-Hydroxyverbindung mit dem Inertgas wird in bekannten Apparaten, beispielsweise in einer Packungskolonne oder Füllkörperkolonne, durchgeführt. Die aromatische mono-Hydroxyverbindung wird durch Kondensieren beispielsweise an einem Wärmetauscher aus dem Inertgasstrom entfernt.

In bevorzugten Ausführungsformen erfolgt die Abtrennung der aromatischen mono-Hydroxyverbindung vom Bis(4-hydroxyaryl)alkan aus der Schmelze des Addukts aus Bis(4-hydroxyaryl)alkan und aromatischer mono-Hydroxyverbindung in wenigstens einem Desorber. Dabei wird die aromatischen mono-Hydroxyverbindung mit dem Inertgasstrom ausgetrieben und damit abgetrennt und das Bis(4-hydroxyaryl)alkan als Sumpfprodukt des Desorbers gewonnen. Als Desorber kommen beliebige, dem Fachmann bekannte Desorptionsapparaturen in Frage. Beispielhaft seien hierzu Röhrenbündeldesorber und Kolonnendesorber (Packungsdesorber) genannt. Erstgenannter enthält vorzugsweise im Wesentlichen Röhrenbündelwärmetauscher. Diese Röhrenbündelwämetauscher sind in bevorzugten Ausführungsformen stehend angeordnet und in ihrem unteren Teil mit einer Anzahl von Düsen ausgestattet, über die das gegebenenfalls vorgeheizte, bevorzugt 160 bis 210 °C heiße Inertgas eingetragen wird. Die Beheizung der Rohbündelwärmetauscher erfolgt durch Rohre und über den äußeren Mantel. Die Zwischenräume zwischen den Wärmeaustauscherrohren sind vorzugsweise produktseitig mit Keramikkugeln (Steatit) gefüllt. Der Produktraum des Desorbers ist zum größten Teil mit Flüssigkeit (Schmelze) gefüllt. Solche Desorber sind beispielsweise in EP-A 1 242 349 beschrieben.

Der aus dem Desorber ausgetragene Inertgasstrom wird gegebenenfalls abgekühlt, bevorzugt auf 30 bis 80°C, und optional einer Filtration unterzogen. Anschließend wird der Inergasstrom in wenigstens einem Kompressor komprimiert, vorzugsweise erfolgt die Komprimierung auf 1,5 bis 4 bar_{absolut} Geeignete Kompressoren sind handelsüblich und dem Fachmann bekannt.

Die Reinigung des im Kreis geführten Inertgases erfolgt durch intensives Waschen mit wenigstens einem Waschmedium. Dieses Waschen kann beispielsweise in einem Gaswäscher erfolgen. Der Waschvorgang kann je nach Bedarf einmal oder mehrmals - im letzteren Fall dann gegebenenfalls in mehreren Gaswäschern - durchgeführt werden. Geeignete Gaswäscher sind dem Fachmann bekannt, wie z.B. Waschtürme. Als Waschmedium geeignet sind Flüssigkeiten, die die aromatischen mono-Hydroxyverbindungen lösen oder binden und somit aus dem Inertgasstrom entfernen können.

In einer bevorzugten Ausführungsform wird bei der Kompression des Inertgases des Kreislaufstroms in einem Kompressor die dort eingesetzte Sperrflüssigkeit als Waschmedium genutzt. Bei der Sperrflüssigkeit handelt es sich um eine Flüssigkeit, die die Schmierung der Gleitringdichtung(en) des oder der Kompressor(en) sicherstellt.

Geeignete Waschmedien sind beispielsweise Wasser und/oder alkalische wässrige Lösungen. Dabei ist im Rahmen der Erfindung unter einer alkalischen wässrigen Lösung eine solche mit einem pH-Wert von 7 oder größer zu verstehen. Als Waschmedium werden bevorzugt Wasser oder eine schwach alkalische wässrige Lösung eingesetzt. Bei dem als Waschmedium verwendeten Wasser handelt es sich vorzugsweise um entmineralisiertes Wasser. Der pH-Wert einer verwendeten alkalischen wässrigen Lösung liegt bevorzugt im Bereich von 7 bis 12, besonders bevorzugt 7,5 bis 11, ganz besonders bevorzugt 8 bis 10. Zur Herstellung einer geeigneten alkalischen wässrigen Lösung können alle basisch wirkenden, wasserlöslichen Substanzen eingesetzt werden. Bevorzugt werden zur Herstellung einer geeigneten alkalischen wässrigen Lösung Alkali- und Erdalkalihydroxide eingesetzt. In einer bevorzugten Ausführungsform des erfindungsgemäßen Verfahrens wird der pH-Wert vor und nach dem Waschen kontrolliert, so dass Über- und Unterdosierungen bei der Reinigung des Inertgasstroms vermieden werden. Der Inertgasstrom kann gegebenenfalls anschließend einer zweiten Wäsche mit einem zweiten Waschmedium zugeführt werden. Das zweite Waschmedium weist dann bevorzugt einen pH-Wert von 5 bis 7 auf. Ein besonders bevorzugtes zweites Waschmedium ist Wasser, ganz besonders bevorzugt entmineralisiertes Wasser.

Unter den angegebenen pH-Werten sind, soweit nicht anders gekennzeichnet, pH-Werte zu verstehen, die bei 25°C bestimmt werden.

Für die Beaufschlagung des oder der der Komprimierung und Reinigung des Inertgasstroms nachgeschalteten Demister(s) können als Flüssigkeiten die gleichen Waschmedien eingesetzt werden, die auch in der Gaswäsche, sowohl der ersten als auch der gegebenenfalls zweiten, verwendet werden. Bauformen für geeignete Demister sind dem Fachmann bekannt und in der Literatur beschrieben, vgl. z.B. A. Bürkholz, E. Muschelknautz, Chemie Ingenieur Technik, 1972, Vol 54, S. 892-900. Vorzugsweise weisen geeignete Demister Drahtgestrick-Packungen aus Metallen oder Kunststoffen auf. Als Metalle eignen sich hierfür beispielsweise Edelstähle, Aluminium, Kupfer, Nickel, als Kunststoffe beispielsweise Polyethylen, Polypropylen oder Polytetrafluorethylen.

Die Beaufschlagung des Demisters mit Flüssigkeit kann im Gleichstrom, d.h. auf der vom Inertgasstrom angeströmten Packungsseite, oder im Gegenstrom, d.h. auf der Packungsseite, aus der der Inertgasstrom austritt, erfolgen. Für die Gegenstrom-Beaufschlagung ist eine senkrechte Anordnung des Demisters unter Anströmen der Packung mit Inertgasstrom von der unteren Seite vorteilhaft. Die Gleichstrom-Beaufschlagung kann bei beliebigen Anordnungen des Demisters erfolgen. Bevorzugt ist daher die Beaufschlagung des Demisters mit Flüssigkeit auf der vom Inertgasstrom angeströmten Packungsseite (Gleichstrom-Beaufschlagung). Die Beaufschlagung des Demisters mit Flüssigkeit geschieht vorzugsweise mit Hilfe von Düsen, besonders bevorzugt Sprühdüsen.

Bevorzugt werden für die Beaufschlagung des Demisters entmineralisiertes Wasser oder verdünnte Natronlauge verwendet. Als verdünnte Natronlauge eignet sich beispielsweise Natronlauge mit einen NaOH-Gehalt von bis zu 10 Gew.-%. Vorzugsweise werden Mengen von 1 Liter eingedüster Flüssigkeit pro 1 - 500 m³ rezykliertem Inertgas, besonders bevorzugt 1 Liter eingedüste Waschlösung pro 30 - 100 m³ rezykliertem Inertgas verwendet.

Durch diese Maßnahme der Beaufschlagung des Demisters mit einer Flüssigkeit kann die Betriebszeit eines Demisters zwischen zwei notwendigen Reinigungsschritten um das 2- bis 500-fache der üblichen Betriebszeit des gleichen Demisters zwischen zwei notwendigen Reinigungsschritten verlängert werden, der nicht mit Waschlösung wie oben beschrieben beaufschlagt wird, ohne dass der kontinuierliche Betrieb unterbrochen werden muss und es zu Produktionsausfällen kommt.

Der so gereinigte Inertgasstrom wird wieder in Schritt a) des erfindungsgemäßen Verfahrens zurückgeführt. So wird das verwendete Inertgas im erfindungsgemäßen Verfahren im Kreis geführt, was das Verfahren besonders effizient und wirtschaftlich macht.

Bevorzugt kann der rezyklierte Inertgasstrom vor der Rückführung in Schritt a) - z.B. vor der Rückführung in einen Desorber - in einem Inertgasvorwärmer vorgewärmt werden. Vorzugsweise erfolgt diese Vorwärmung auf Temperaturen von 150 bis 250°C, besonders bevorzugt 170 bis 220°C. Als Inertgasvorwärmer eignen sich beispielsweise Wärmetauscher, wie z.B. Rohrbündelwärmetauscher oder Plattenwärmetauscher. Geeignete Wärmetauscher sind in der Literatur beschrieben und dem Fachmann bekannt.

Durch das erfindungsgemäße Verfahren werden Ablagerungen und damit Verstopfungen der gegebenenfalls verwendeten Inertgasvorwärmer deutlich reduziert bzw. nahezu vollständig vermieden und damit die Zahl erforderlicher Reinigungen, die eine Unterbrechung des kontinuierlichen Betriebes erfordern und Produktionsausfälle nach sich ziehen, signifikant verringert.

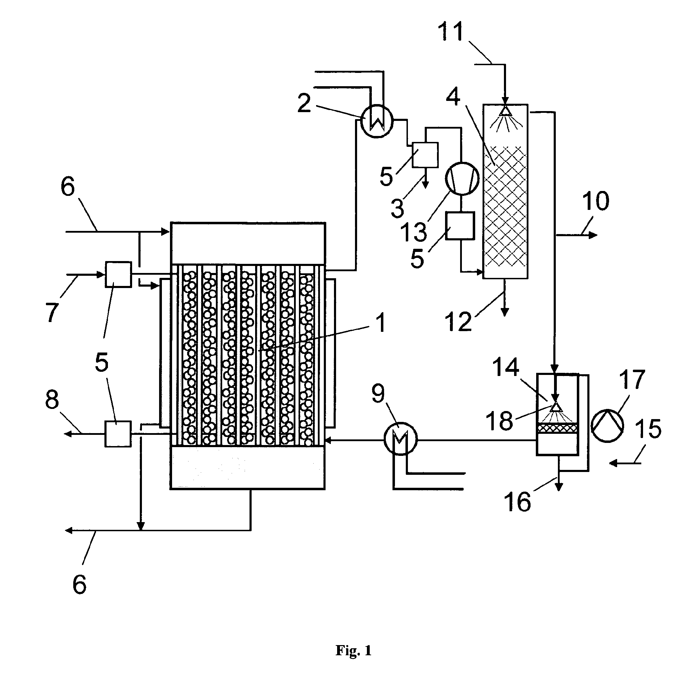

Fig. 1 beschreibt beispielhaft eine besonders bevorzugte Ausführungsform des erfindungsgemäßen Verfahrens, ohne jedoch limitierend verstanden zu werden. Darin bedeuten:
- 1: Desorber
- 2: Kühler
- 3: Ablauf aromatische mono-Hydroxyverbindung
- 4: Gas-Waschturm (Gaswäscher)
- 5: Filter
- 6: Beheizung
- 7: Addukteinleiteeinheit
- 8: Bis(4-hydroxyaryl)alkan-Produktentnahmeeinheit
- 9: Inertgaserhitzer (Wärmetauscher)
- 10: Gas-Ausspeisung
- 11: Einspeisung Gaswaschwasser
- 12: Ausspeisung Gaswaschwasser
- 13: Kreisgaskompressor
- 14: Demister
- 15: Einspeisung Demisterwaschflüssigkeit
- 16: Ausspeisung Demisterwaschflüssigkeit
- 17: Pumpe Demisterwaschflüssigkeit
- 18: Sprühdüse

Durch die Addukteinleiteeinheit (7) wird das Addukt aus Bis(4-hydroxyaryl)alkan und aromatischer mono-Hydroxyverbindung, bevorzugt aus Bisphenol A und Phenol, durch einen Filter (5) dem Desorber (1) zugeleitet. Dabei handelt es sich bei dem Desorber um einen Röhrenbündelwämetauscher, dessen Zwischenräume zwischen den Wärmeaustauscherrohren produktseitig mit Keramikkugeln (Steatit) gefüllt sind. Der Desorber wird zur Aufschmelzung des Adduktes durch die Beheizung (6) beheizt und dass im unteren Teil (Sumpf) gewonnene Bis(4-hydroxyaryl)alkan, bevorzugt Bisphenol A, über einen Filter (5) über die Produktentnahmeeinheit (8) aus dem Desorber ausgetragen. Über einen Inertgaserhitzer (9) wird das Inertgas im unteren Teil in den Desorber ein und durch die Adduktschmelze geleitet. Der mit der aromatischen mono-Hydroxyverbindung, bevorzugt Phenol, beladene Inertgasstrom wird am oberen Teil aus dem Desorber ausgeleitet, in einem Kühler (2) abgekühlt und durch einen Filter (5) geleitet. Bei der Abkühlung wird der größte Teil der im Inertgasstrom enthaltenen aromatischen mono-Hydroxyverbindung auskondensiert. Die dabei aus dem Inertgas entfernte aromatische mono-Hydroxyverbindung, bevorzugt Phenol, wird über einen Ablauf (3) abgeführt. Der Inertgasstrom wird anschließend im Kompressor (13) komprimiert und durch einen weiteren Filter (5) in den unteren Teil eines Gas-Waschturm geleitet, in den über eine Einspeisung (11) Gaswaschwasser eingeleitet und über eine Ausspeisung (12) mit Verunreinigungen, z.B. aromatischer mono-Hydroxyverbindung, beladenes Gaswaschwasser ausgeleitet wird. Das so gewaschene Inertgas wird im oberen Teil des Gas-Waschturms (4) ausgetragen und durch den Demister (14) geleitet, der mittels einer Sprühdüse (18) auf der vom Inertgasstrom angeströmten Seite mit Flüssigkeit (Demisterwaschflüssigkeit) beaufschlagt, d.h. besprüht, wird. Dabei wird unbeladene Demisterwaschflüssigkeit über eine Einspeisung (15) von außen zugeführt und über eine Pumpe (17) in die Düse (18) geleitet und die mit Verunreinigungen, z.B. aromatischer mono-Hydroxyverbindung, beladene Demisterwaschflüssigkeit über eine Ausspeisung (16) ganz oder teilweise abgeführt. Die Demisterwaschflüssigkeit kann im Rahmen der Erfindung entweder vollständig durch frische, unbeladene Waschflüssigkeit über die Einspeisung (15) ersetzt und die beladene vollständig über die Ausspeisung (16) abgeführt werden. Es ist aber auch möglich einen Teil der beladenen Demisterwaschflüssigkeit über die Einspeisung (15) mit zugeführter frischer, unbeladener Waschflüssigkeit zu vermischen und so teilweise mehrfach zu verwenden. Dabei wird über die Ausspeisung (16) lediglich ein Teil der beladenen Waschflüssigkeit abgeführt. Über die Gasausspeisung (10) werden gasförmige Nebenkomponenten aus dem Kreislauf abgeführt und so verhindert, dass sich solche Nebenkomponenten über eine bestimmte Konzentration hinaus anreichern.

Die nach dem erfindungsgemäßen Verfahren hergestellten Bis(4-hydroxyaryl)alkane zeichnen sich durch eine sehr gute Eigenfarbe sowie hohe Reinheit aus, insbesondere weisen sie geringe Gehalte an aromatischen mono-Hydroxyverbindungen von bevorzugt weniger als 100 ppm, besonders bevorzugt weniger als 50 ppm auf sowie geringen Gehalten an Zersetzungsprodukten, wie z.B. Isopropenylphenol, dimeres Isopropenylphenol, auf.

Im Rahmen der vorliegenden Erfindung sind unter ppm - soweit nichts anderes angegeben - Gewichtsteile zu verstehen.

Aus den nach dem erfindungsgemäßen Verfahren hergestellten Bis(4-hydroxyaryl)alkanen können Polymere wie Polycarbonate oder Epoxidharze mit geringer Eigenfarbe hergestellt werden.

Die folgenden Beispiele dienen der beispielhaften Erläuterung der Erfindung und sind nicht als Beschränkung aufzufassen.

### Beispiele

### Beispiel 1

39 t/h Schmelzegemisch BPA/Phenol (90/10 Gew.-%) werden parallel in 5 Desorber eingebracht. In die Desorber werden insgesamt kontinuierlich 5840 m³ Stickstoff/h (Kreislaufstrom) eingeleitet. Das Phenol wird vom Stickstoff aufgenommen und anschließend mittels mehrerer Wärmetauscher kondensiert. Der Stickstoff wird anschließend drei Kompressoren, deren Sperrflüssigkeit mit verdünnter NaOH-Lauge (pH-Wert 10) beaufschlagt wird, zugeführt, verdichtet und in einen Gaswäscher, der mit entmineralisiertem Wasser betrieben wird, eingeleitet. Der so behandelte Stickstoff wird durch einen Demister aus einer Metallpackung (Edelstahlgeflechtpackung aus Edelstahl 1.4571) geleitet, wobei auf der angeströmten Seite des Demisters 100 L/h entmineralisiertes Wasser über eine Vollkegeldüse flächig auf den Demister verteilt werden. Danach wird das Inertgas nach Vorwärmung auf 190 °C in den Wärmetauschern (ein Stickstoffvorwärmer pro Desorber) wieder in den Desorber zurückgeführt und zur Phenolstrippung eingesetzt. Man erhält so eine phenolarme (Phenolwert 40 ppm) farbhelle BPA-Schmelze mit einer Schmelzefarbzahl von 8 Hazen. Die Betriebszeit des Demisters beträgt mehr als 3 Monate, Ablagerungen an den Stickstoffvorwärmern werden fast vollständig vermieden.

### Vergleichsbeispiel 1

Die Durchführung erfolgt wie in Beispiel 1, mit dem Unterschied, dass auf die Eindüsung von Wasser auf die angeströmte Seite des Demisters verzichtet wird. Die Betriebszeit des Demisters beträgt infolge von Feststoffablagerungen auf der Metallpackung weniger als 1 Tag; Ablagerungen an den Stickstoffvorwärmern führen nach ca. 12 Monaten zu Teilverstopfungen, so dass die Vorwärmer außer Betrieb genommen und gereinigt werden müssen.

## Patentansprüche

1. Verfahren zur Herstellung von Bis(4-hydroxyaryl)alkanen aus Addukten von Bis(4-hydroxyaryl)alkanen und aromatischen mono-Hydroxyverbindungen, **dadurch gekennzeichnet, dass**
a) durch eine Schmelze eines Addukts aus einem Bis(4-hydroxyaryl)-alkan und einer aromatischen mono-Hydroxyverbindung bei 150°C bis 230°C ein Inertgas geleitet wird, wobei der Inertgasstrom die aromatische mono-Hydroxyverbindung aus der Schmelze entfernt,
b) die aromatische mono-Hydroxyverbindung aus dem Inertgasstrom durch Kondensation entfernt wird,
c) der Inertgasstrom anschließend
aa) komprimiert, gereinigt und
bb) durch wenigstens einen Demister geleitet wird, der mit einer Flüssigkeit beaufschlagt wird, und
cc) wieder in Schritt a) zurückgeführt wird.

2. Verfahren nach Anspruch 1, **dadurch gekennzeichnet, dass** bei dem die in Unterschritt bb) von Schritt c) verwendete Flüssigkeit Wasser oder eine alkalische wässrige Lösung ist.

3. Verfahren nach Anspruch 1 oder 2, **dadurch gekennzeichnet, dass** die in Unterschritt bb) von Schritt c) verwendete Flüssigkeit einen pH-Wert im Bereich von 7 bis 12 hat.

4. Verfahren nach wenigstens einem der Ansprüche 1 bis 3, **dadurch gekennzeichnet, dass** die in Unterschritt bb) von Schritt c) verwendete Flüssigkeit in Mengen von 1 Liter Flüssigkeit pro 1 - 500 m³ Inertgas eingesetzt wird.

5. Verfahren nach wenigstens einem der Ansprüche 1 bis 4, **dadurch gekennzeichnet, dass** der Demister in Unterschritt bb) von Schritt c) auf der angeströmten Seite mit der Flüssigkeit beaufschlagt wird.

6. Verfahren nach wenigstens einem der Ansprüche 1 bis 5, **dadurch gekennzeichnet, dass** der Inertgasstrom vor Zurückführen in Schritt a) vorgewärmt wird.

7. Verfahren nach wenigstens einem der Ansprüche 1 bis 6, **dadurch gekennzeichnet, dass** Bis(4-hydroxyaryl)alkane solche der allgemeinen Formel (I) sind, 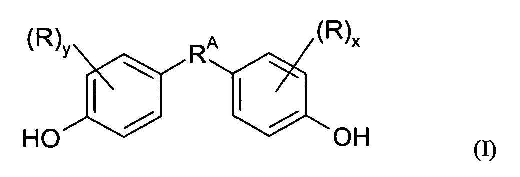 worin
R^{A} für einen linearen oder verzweigten C₁-C₁₈-Alkylenrest, bevorzugt C₁-C₆-Alkylenrest, oder einen C₅-C₁₈-Cycloalkylenrest, bevorzugt einen C₅-C₁₂-Cycloalkylenrest steht,
R unabhängig voneinander für einen linearen oder verzweigten C₁-C₁₈-Alkylrest, bevorzugt C₁-C₆-Alkylrest, einen C₅-C₁₈-Cycloalkylrest, bevorzugt einen C₅-C₁₂-Cycloalkylrest, einen C₆-C₂₄-Arylrest, bevorzugt einen C₆-C₁₂-Arylrest, oder einen Halogenrest stehen und
x und y unabhängig voneinander für 0 oder eine ganze Zahl von 1 bis 4, bevorzugt unabhängig voneinander für 0, 1 oder 2 stehen.

8. Verfahren nach wenigstens einem der Ansprüche 1 bis 7, **dadurch gekennzeichnet, dass** aromatische mono-Hydroxyverbindungen solche der allgemeinen Formel (II) sind, 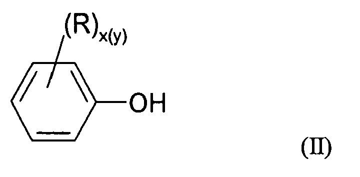 die in p-Position nicht substituiert sind und worin,
R unabhängig voneinander für einen linearen oder verzweigten C₁-C₁₈-Alkylrest, bevorzugt C₁-C₆-Alkylrest, einen C₅-C₁₈-Cycloalkylrest, bevorzugt einen C₅-C₁₂-Cycloalkylrest, einen C₆-C₂₄-Arylrest, bevorzugt einen C₆-C₁₂-Arylrest, oder einen Halogenrest stehen und
x oder y für 0 oder eine ganze Zahl von 1 bis 4, bevorzugt für 0, 1 oder 2 stehen.

9. Verfahren nach wenigstens einem der Ansprüche 1 bis 8, **dadurch gekennzeichnet, dass** als Addukts aus einem Bis(4-hydroxyaryl)-alkan und einer aromatischen mono-Hydroxyverbindung das Addukt von Bisphenol A (2,2-Bis-(4-hydroxyphenyl)-propan) und Phenol eingesetzt wird.

10. Bis(4-hydroxyaryl)-alkan mit einem Gehalt an aromatischen mono-Hydroxyverbindungen von weniger als 100 ppm, erhältlich durch ein Verfahren gemäß wenigstens einem der Ansprüche 1 bis 9.
